# EUROPÄISCHE PATENTSCHRIFT

(11) **EP 0 401 652 B1**
(45) Veröffentlichungstag und Bekanntmachung des Hinweises auf die Patenterteilung: **17.08.1994**
(21) Anmeldenummer: 90110206.1
(22) Anmeldetag: 30.05.1990
(51) Int. Cl.: C07D 295/08, A61K 31/495

(54) **Hydroxylierte 1-Phenyl-4-[3-(naphth-1-yl-oxy)-2-hydroxypropyl]-piperazine, Verfahren zu ihrer Herstellung sowie Arzneimittel, die diese Verbindungen enthalten**
Hydroxylated 1-phenyl-4-[3-(naphth-1-yloxy)-2-hydroxypropyl] piperazines, process for their preparation as well as medicines containing these compounds
Pipérazines 1-phényl-4-[3-(naphth-1-yloxy)-2-hydroxypropyliques] hydroxylées, procédé pour leur préparation et médicaments contenant ces composés

(30) Priorität: 07.06.1989 DE 3918542
(43) Veröffentlichungstag der Anmeldung: 12.12.1990
(73) Patentinhaber: BOEHRINGER MANNHEIM GMBH, 68298 Mannheim (DE)
(72) Erfinder: Sponer, Gisbert, Dr. med. vet., D-6941 Laudenbach (DE); Borbe-Vomer, Harald O., Dr. rer. nat., D-6500 Mainz 32 (DE); Engel, Jürgen, Prof. Dr., D-8755 Alzenau (DE); Kutscher, Bernhard, Dr. phil. nat., D-6457 Maintal 1 (DE); Niebch, Georg, Dr. rer. nat., D-6458 Rodenbach (DE); Siebert-Weigel, Marianne, Dr. med., D-6000 Frankfurt 1 (DE)

(56) Entgegenhaltungen:
- EP-A- 0 089 634
- DE-A- 2 155 697
- DE-A- 2 235 597
- DE-A- 2 408 804

## Beschreibung

Gegenstand der Erfindung sind Verbindungen der Formel I 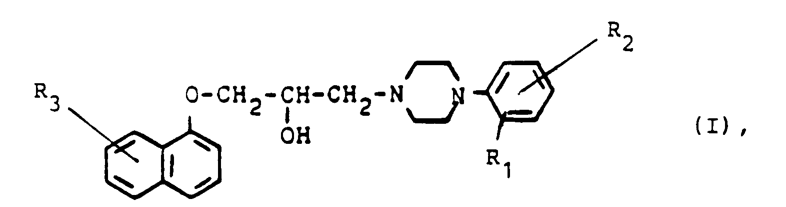
in der
- R₁: eine Methoxy- oder Hydroxylgruppe,
- R₂: Wasserstoff oder eine Hydroxylgruppe und
- R₃: Wasserstoff oder eine Hydroxylgruppe
bedeuten, mit der Maßgabe, daß R₂ und R₃ nicht gleichzeitig Wasserstoff bedeuten, wenn R₁ eine Methoxygruppe darstellt,
sowie deren pharmakologisch verträgliche Salze, Verfahren zu ihrer Herstellung sowie Arzneimittel, die diese Verbindungen enthalten.

Aus DE-B-24 08 804 ist die Verbindung 1-(2-Methoxyphenyl)-4-[3-(naphth-1-yl-oxy)-2-hydroxy-propyl]-piperazin bekannt, die antihypertensive Eigenschaften besitzt und durch Dextran ausgelöste anaphylaktoide Reaktionen bei Ratten hemmt.

Die erfinderischen Verbindungen zeigen ebenfalls ausgeprägte antihypertensive Eigenschaften. Darüber hinaus können sie in der Therapie der Dysurie bei einer Prostata-Hypertrophie eingesetzt werden. Sie sind daher wertvolle Arzneimittel zur Behandlung dieser Krankheiten.

Bevorzugte Verbindungen der Formel I sind:
1-(2-Methoxy-4-hydroxy-phenyl)-4-[3-(naphth-1-yl-oxy)-2-hydroxy-propyl]-piperazin,
1-(2-Methoxyphenyl)-4-[3-(4-hydroxynaphth-1-yl-oxy)-2-hydroxy-propyl]-piperazin und
1-(2-Hydroxyphenyl)-4-[3-(naphth-1-yl-oxy)-2-hydroxy-propyl]-piperazin.

Der Substituent R₃ kann in sämtlichen Positionen des Naphthylringes stehen, bevorzugt ist die 4-Stellung.

Die erfindungsgemäßen Verbindungen können in an sich bekannter Weise wie folgt hergestellt werden, in dem man
a) eine Verbindung der Formel II 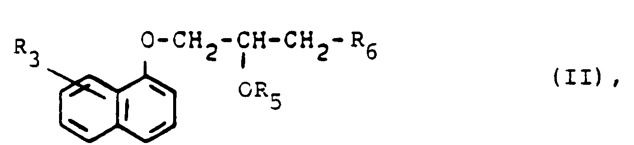 in der
   - R₃: Wasserstoff oder eine gegebenenfalls geschützte Hydroxygruppe bedeuten,
   - R₅: Wasserstoff und R₆ Halogen darstellen, wobei die Reste R₅ und R₆ auch zusammen einen Valenzstrich bedeuten können,
   mit einer Verbindung der Formel III 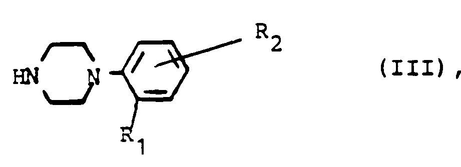 in der
   - R₁ und R₂: die oben angegebene Bedeutungen haben,
   umsetzt, oder
b) eine Verbindung der Formel IV 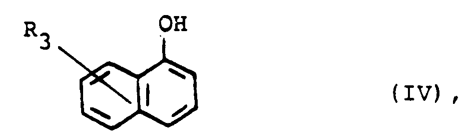 in der
   - R₃: Wasserstoff oder eine gegebenenfalls geschützte Hydroxygruppe darstellt,
   mit einer Verbindung der Formel V 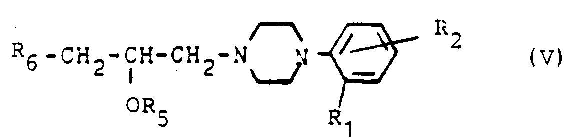 in der
   - R₁ und R₂: die oben angegebenen Bedeutungen haben,
   - R₅: Wasserstoff und R₆ Halogen darstellen,
   wobei R₅ und R₆ zusammen auch einen Valenzstrich bedeuten können,
   umsetzt
   und anschließend gegebenenfalls die Hydroxylschutzgruppe abspaltet und die so erhaltenen Verbindungen in ihre pharmakologisch verträglichen Salze überführt.
   Als Halogen des Restes R₆ kommt bevorzugt Chlor in Frage.
   Die Hydroxylschutzgruppe ist bevorzugt Benzyl.

Die Umsetzung erfolgt vorzugsweise durch Zusammenfügen äquimolarer Mengen der Reaktionskomponente unter Zusatz von Lösungsmitteln, wie z.B. Methanol, Ethanol, Isopropanol,und anschließendem Erhitzen unter Rückfluß.

Die Ausgangsverbindungen der Formel II erhält man durch Umsetzung von 1-Naphthol-Derivaten mit Epichlorhydrin, wobei bei Dihydroxy-Derivaten die zweite OH-Gruppe durch eine Benzylgruppe geschützt wird, die nach der Umsetzung mit Verbindungen der Formel III in üblicher Weise abgespalten wird, z.B. durch Hydrieren mit Pd/C.

Die Verbindungen der Formel V werden in analoger Weise durch Umsetzung von bekannten entsprechenden N-Phenyl-Piperazinen mit Epichlorhydrin erhalten. Die weitere Umsetzung mit 1-Naphthol-Derivaten erfolgt wiederum in Gegenwart von Alkoholen unter Rückfluß.

Zur Herstellung von Salzen setzt man die erfindungsgemäße Verbindung mit pharmakologisch verträglichen organischen oder anorganischen Säuren, z.B. Salzsäure, Schwefelsäure, Phosphorsäure, Milchsäure, Zitronensäure oder Alkylsulfonsäure, um.

Zur Herstellung von Arzneimitteln werden die erfindungsgemäßen Substanzen oder deren Salze in an sich bekannter Weise mit geeigneten pharmazeutischen Trägersubstanzen, Aroma-, Geschmacks- und Farbstoffen gemischt und beispielsweise als Tabletten oder Dragées ausgeformt oder unter Zugabe entsprechender Hilfsstoffe in Wasser oder Öl, wie z.B. Olivenöl, suspendiert oder gelöst.

Die Dosierung der erfindungsgemäßen Substanzen hängt von der Art der Erkrankung sowie dem Alter der Patienten ab. Üblicherweise werden bei der oralen Therapie Tabletten mit einer Wirkstoffmenge von 10 bis 100 mg 1 bis 3 mal täglich appliziert.

In den nachfolgenden Beispielen wird die Herstellung einiger erfindungsgemäßer Verbindungen beschrieben.

### Beispiel 1

### 1-(2-Methoxy-4-hydroxyphenyl)-4-[3-(naphth-1-yl-oxy)-2-hydroxy-propyl]-piperazin

4,56 g (0,025 Mol) 1-(2-Hydroxyphenyl)-piperazin und 5,6 g (0,028 Mol) 2,3-Epoxypropyl-naphthylether werden in 50 ml 2-Propanol gelöst. Die Lösung wird 5 Stunden unter Rühren und Rückfluß erhitzt. Danach wird mit weiteren 50 ml 2-Propanol verdünnt und mit 2-propanolischer Salzsäure angesäuert. Es fällt ein voluminoses Produkt aus, das abgesaugt, mehrmals mit Isopropanol gewaschen und aus Methanol umkristallisiert wird.
Ausbeute: 8,8 g 77 % d.Th. Fp. 249-250° C

### Beispiel 2

### 1-(2-Methoxy-4-hydroxyphenyl)-4-[3-(naphth-1-yl-oxy)-2-hydroxy-propyl]-piperazin

Die Lösung aus 4,16 g (0,02 Mol) 1-(2-Methoxy-4-hydroxyphenyl)-piperazin und 4,2 g (0,021 Mol) 2,3-Epoxypropylnaphthylether in 50 ml Isopropanol wird 4 Stunden unter Rühren und Rückfluß erhitzt. Die Lösung wird eingeengt, der teerartige Rückstand in Methanol gelöst. Es wird mit isopropanolischer Salzsäure angesäuert und vorsichtig Ether zugefügt. Es fällt ein öliges Produkt aus. Von diesem wird abgegossen. Die Lösung wird mit Aceton verdünnt. Nach kurzer Zeit beginnt Substanz auszukristallisieren. Nach beendeter Kristallisation wird abgesaugt und erneut aus Methanol/Ether umkristallisiert.
Ausbeute: 4,8 g 50 % d.Th. Fp. 230-232° C

### Beispiel 3

### 1-(2-Methoxyphenyl)-4-[3-(4-hydroxynaphth-1-yl-oxy)-2-hydroxy-propyl]-piperazin

a) 1-Benzyloxy-4-hydroxynaphthalin
   23,7 g (0,147 Mol) 1,4-Dihydroxynaphthalin (durch Ausschütteln der etherischen Lösung mit Na-dithionitlösung gereinigt!) werden in 200 ml Aceton gelöst. Nach Zufügen von 20,3 g (0,147 Mol) pulverisierter Pottasche und Erhitzen auf Rückflußtemperatur werden im Laufe von 60 Min. 18,6 g (16,9 ml) Benzylchlorid zugetropft. Danach wird noch 4 Stunden unter Rühren und Rückfluß erhitzt, eingeengt, der Rückstand mit 100 ml Wasser versetzt und mit Ether extrahiert. Die Etherlösung wird mit MgSO₄ getrocknet, filtriert und eingeengt. Der teerartige Rückstand wird mit Cyclohexan extrahiert. Die vereinten Auszüge werden abgekühlt, wobei zuerst erhebliche Mengen teerartiges Produkt ausgeschieden werden. Danach kristallisiert die reine Substanz aus. Sie wird mit Cyclohexan gewaschen und getrocknet.
   Ausbeute: 4,3 g 11,9 % d.Th. Fp. 118-119°C
b) 1-Benzyloxy-4-(2,3-epoxypropyl)-naphthylether
   Zu einer Lösung von 4,3 g (0,017 Mol) 1-Hydroxy-4-benzyloxynaphthalin in 15 ml (0,19 Mol) Epichlorhydrin, die unter Rühren auf ihren Siedepunkt erhitzt wird, tropfen vorsichtig 3,7 ml 5 m NaOH (0,0187 Mol) zu. Die Apparatur ist mit einem Wasserabscheider versehen, so daß das Wasser mit Epichlorhydrin azeotrop aus dem Reaktionsgemisch abdestilliert wird. Nach Abdestillieren der theoretischen Wassermenge wird noch eine Stunde unter Rühren und Rückfluß erhitzt, danach mit 50 ml Toluol versetzt, von anorganischen Bestandteilen abfiltriert und die Lösung am Rotationsverdampfer eingeengt. Die Substanz wird ohne weitere Reinigung für die nächste Reaktion eingesetzt.
   Ausbeute: 6,1 g
c) 1-(2-Methoxyphenyl)-4-[3-(4-benzyloxy-naphth-1-yl-oxy)-2-hydroxy-propyl]-piperazin
   Eine Lösung von 5,2 g (0,017 Mol) 1-Benzyloxy-4-(2,3-epoxy-propyl)-naphthylether und 8,3 g (0,034 Mol) 1-(2-Methoxyphenyl) piperazin in 50 ml Isopropanol wird unter Zusatz einer geringen Wassermenge ca. 5 Stunden unter Rühren und Rückfluß erhitzt. Danach wird eingeengt und der ölige Rückstand säulenchromatografisch mit Kieselgel (Geduran S, 60; Elutionsmittel: 90 CH₂Cl₂, 10 CH₃OH, 1 NH₃ 25% wäßrig) gereinigt. Nach Einengen der gesammelten Eluate wird der Rückstand in Methanol gelöst, mit isopropanolischer Salzsäure angesäuert und mit Ether bis zur beginnenden Trübung versetzt. Das auskristallisierte Produkt wird abgesaugt, mit Aceton gewaschen und erneut aus Methanol/Ether umkristallisiert.
   Ausbeute: 4,8 g 56 % d.Th. Fp. 143-145°C
d) 1-(2-Methoxyphenyl)-4-[3-(4-hydroxy-naphth-1-yl-oxy)-2-hydroxy-propyl]-piperazin
   2,8 g (0,005 Mol) Benzyloxyverbindung werden in 50 ml Methanol in Gegenwart von Pd/C 10 % bei 45 - 50° und einem Wasserstoffdruck einer 60 cm hohen Wassersäule hydriert. Nach etwa 2 Stunden ist die Wasserstoffaufnahme beendet. Die entstandene Substanz ist schwer löslich, so daß sie schon während ihres Entstehens aus der Lösung kristallisiert. Es werden etwa 150 ml Methanol hinzugefügt, unter Rühren wird zum Sieden erhitzt und der Katalysator durch Heißfiltration entfernt. Danach wird im Vakuum weitgehend eingeengt, das Kristallisat abgesaugt, mit Methanol gewaschen und getrocknet.
   Ausbeute: 2,3 g 95 % d.Th. Fp. 225-226°C

## Patentansprüche

1. Verbindungen der Formel I 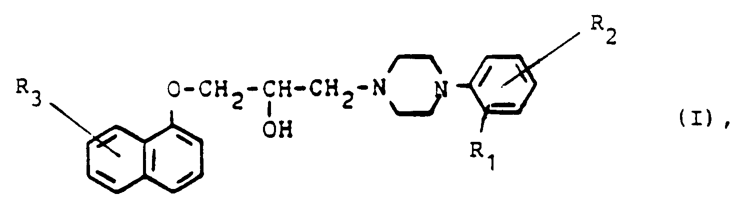 in der
R₁ eine Methoxy- oder Hydroxylgruppe,
R₂ Wasserstoff oder eine Hydroxylgruppe und
R₃ Wasserstoff oder eine Hydroxylgruppe
bedeuten, mit der Maßgabe, daß R₂ und R₃ nicht gleichzeitig Wasserstoff bedeuten, wenn R₁ eine Methoxygruppe darstellt, sowie deren pharmakologisch verträgliche Salze.

2. Verfahren zur Herstellung von Verbindungen der Formel I 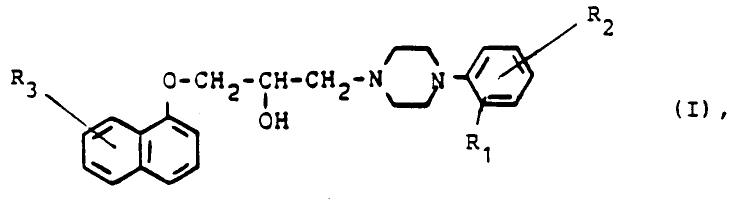 in der
R₁ eine Methoxy- oder Hydroxylgruppe,
R₂ Wasserstoff oder eine Hydroxylgruppe und
R₃ Wasserstoff oder eine Hydroxylgruppe
bedeuten, mit der Maßgabe, daß R₂ und R₃ nicht gleichzeitig Wasserstoff bedeuten, wenn R₁ eine Methoxygruppe darstellt, sowie deren pharmakologisch verträgliche Salze,
dadurch gekennzeichnet, daß man in an sich bekannter Weise eine Verbindung der Formel II
a) 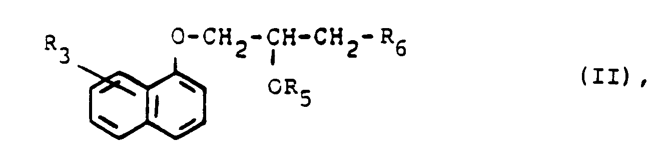 in der
R₃ Wasserstoff oder eine gegebenenfalls geschützte Hydroxygruppe bedeuten,
R₅ Wasserstoff und R₆ Halogen darstellen, wobei die Reste R₅ und R₆ auch zusammen einen Valenzstrich bedeuten könne
mit einer Verbindung der Formel III 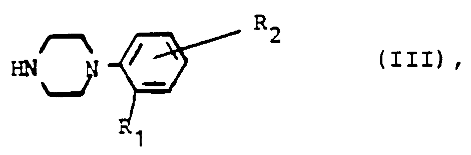 in der
R₁ und R₂ die oben angegebene Bedeutungen haben,
umsetzt, oder
b) eine Verbindung der Formel IV 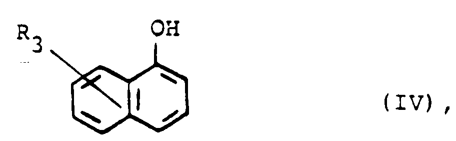 in der
R₃ Wasserstoff oder eine gegebenenfalls geschützte Hydroxygruppe darstellt,
mit einer Verbindung der Formel V 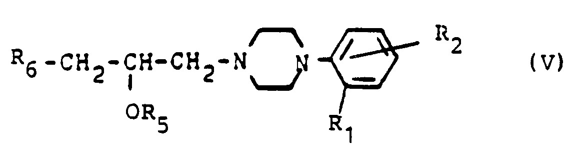 in der
R₁ und R₂ die oben angegebenen Bedeutungen haben,
R₅ Wasserstoff und R₆ Halogen darstellen,
wobei R₅ und R₆ zusammen auch einen Valenzstrich bedeuten können,
umsetzt
und anschließend gegebenenfalls die Hydroxylschutzgruppe abspaltet und die so erhaltenen Verbindungen in ihre pharmakologisch verträglichen Salze überführt.

3. Arzneimittel, enthaltend eine Verbindung gemäß Anspruch 1 neben üblichen Träger- und Hilfsstoffen.

4. Verwendung von Verbindungen gemäß Anspruch 1 zur Herstellung von Arzneimitteln mit antihypertensiven Wirkung.

5. Verwendung von Verbindungen gemäß Anspruch 1 zur Herstellung von Arzneimitteln zur Therapie der Dysurie bei einer Prostata-Hypertrophie.

## Claims

1. Compounds of the formula I 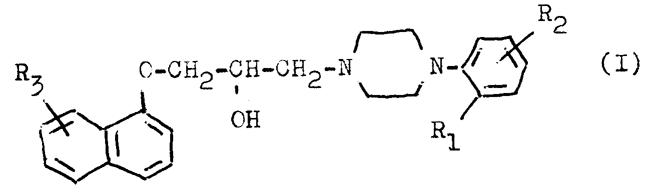 in which R₁ signifies a methoxy or hydroxyl group, R₂ hydrogen or a hydroxyl group and R₃ hydrogen or a hydroxyl group, with the proviso that R₂ and R₃ do not simultaneously signify hydrogen when R₁ represents a methoxy group, as well as their pharmacologcially compatible salts.

2. Process for the preparation of compounds of the formula I 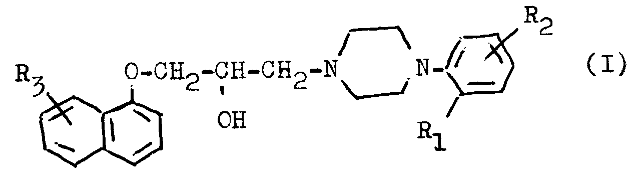 in which R₁ signifies a methoxy or hydroxyl group, R₂ hydrogen or a hydroxyl group and R₃ hydrogen or a hydroxyl group, with the proviso that R₂ and R₃ do not simultaneously signify hydrogen when R₁ represents a methoxy group, as well as of their pharmacologically compatible salts, characterised in that, in per so known manner, one
a) reacts a compound of the formula II 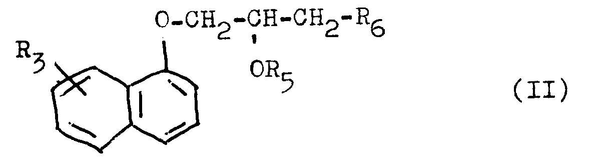 in which R₃ signifies hydrogen or a possibly protected hydroxyl group, R₅ represents hydrogen and R₆ halogen, whereby the radicals R₅ and R₆ can together also signify a valency bond, with a compound of the formula III 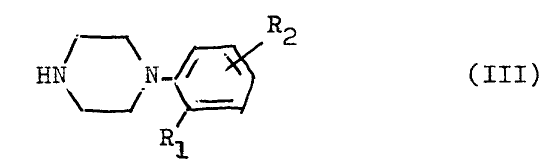 in which R₁ and R₂ have the above-given meanings; or
b) reacts a compound of the formula IV 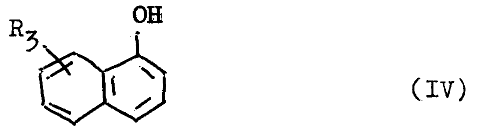 in which R₃ represents hydrogen or a possibly protected hydroxyl group, with a compound of the formula V 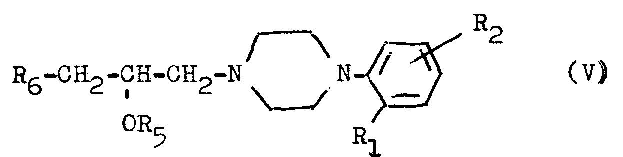 in which R₁ and R₂ have the above-given meanings, R₅ represents hydrogen and R₆ halogen, whereby R₅ and R₆ can together also signify a valency bond; and subsequently possibly splits off the hydroxyl protective group and converts the so-obtained compounds into their pharmacologically compatible salts.

3. Medicaments containing a compound according to claim 1, besides usual carrier and adjuvant materials.

4. Use of compounds according to claim 1 for the preparation of medicaments with antihypertensive action.

5. Use of compounds according to claim 1 for the preparation of medicaments for the therapy of dysuria in the case of a prostatic hypertrophy.

## Revendications

1. Composés de formule I 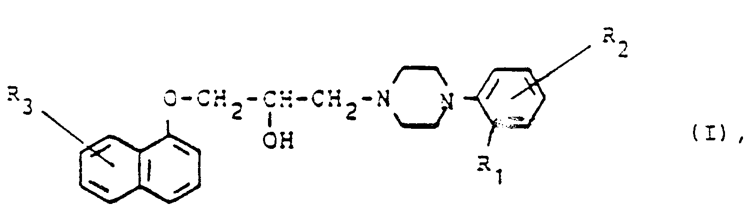 dans laquelle
R₁ représente un groupe méthoxy ou hydroxyle
R₂ un atome d'hydrogène ou un groupe hydroxyle, et
R₃ un atome d'hydrogène ou un groupe hydroxyle, sous reserve que R₂ et R₃ ne représentent pas en même temps un atome d'hydrogène, lorsque R₁ représente un groupe méthoxy,
ainsi que leurs sels pharmacologiquement acceptables.

2. Procédé de préparation de composés de formule I 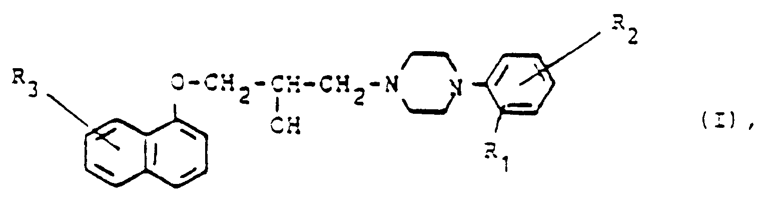 dans laquelle
R₁ représente un groupe méthoxy ou hydroxyle
R₂ un atome d'hydrogène ou un groupe hydroxyle, et
R₃ un atome d'hydrogène ou un groupe hydroxyle, sous réserve que R₂ et R₃ ne représentent pas en même temps un atome d'hydrogène lorsque R₁ représente un groupe méthoxy,
ainsi que leurs sels pharmacologiquement acceptables, caractérisé en ce qu'on fait réagir, de manière en soi connue,
a) un composé de formule II, 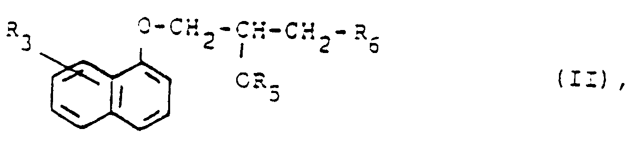 dans laquelle
R₃ représente un atome d'hydrogène ou un groupe hydroxy le cas échéant protégé,
R₅ un atome d'hydrogène et R₆ un atome d'halogène, les restes R₅ et R₆ pouvant représenter également ensemble un trait de valence,
avec un composé de formule III 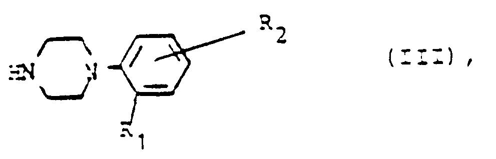 dans laquelle R₁ et R₂ ont les significations données ci-dessus, ou
b) un composé de formule IV 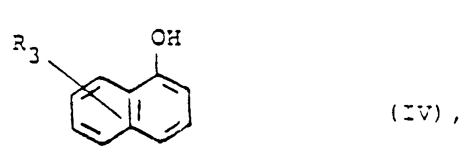 dans laquelle
R₃ représente un atome d'hydrogène ou un groupe hydroxy éventuellement protégé,
avec un composé de formule V 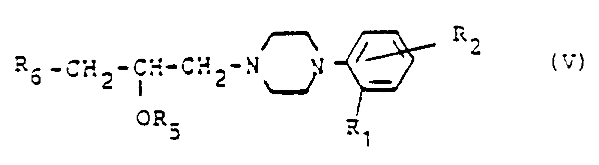 dans laquelle
R₁ et R₂ présentent les significations données ci-dessus,
R₅ représente un atome d'hydrogène et R₆ un atome d'halogène,
R₅ et R₆ pouvant représenter ensemble également un trait de valence
et qu'on élimine ensuite le cas échéant le groupe de protection hydroxyle et que les composés ainsi obtenus sont transformés en leurs sels pharmacologiquement acceptables.

3. Médicaments comprenant un composé selon la revendication 1 avec les véhicules et excipients usuels.

4. Utilisation de composés selon la revendication 1, pour préparer des médicaments ayant un effet anti-hypertensif.

5. Utilisation de composés selon la revendication 1, pour la préparation de médicaments pour la thérapie de dysurie par hypertrophie de la prostate.
